# EUROPEAN PATENT APPLICATION

(11) **EP 2 548 849 A1**
(43) Date of publication of application: **23.01.2013**
(21) Application number: 12176864.2
(22) Date of filing: 18.07.2012
(51) Int. Cl.: C02F 3/00, G01N 33/00, G01N 27/62, C02F 3/28, C02F 11/04

(54) **Process for controlling and monitoring the production of biogas**

(30) Priority: 22.07.2011 LU 91846
(71) Applicant: Centre de Recherche Public - Gabriel Lippmann, 4422 Belvaux (LU)
(72) Inventor: Orlewski, Pierre, 9021 Ettelbruck (LU); Delfosse, Philippe, 3240 Bettembourg (LU); Camara, Malick, 4422 Belvaux (LU)
(74) Representative: Office Freylinger

(57) **Abstract**

The present invention relates to a process for online and direct control of production of biogas in an anaerobic digester comprising the steps of:
a) feeding the anaerobic digester with organic substrate,
b) extracting a sample of biogas from the headspace of the anaerobic digester,
c) analyzing the composition of the sample of biogas and detecting the quantity of one or more volatile fatty acids in said sample of biogas,
d) controlling feeding of the anaerobic digester in view of the quantity of the one or more volatile fatty acids detected in said sample of biogas.

## Description

### Technical field

The present invention generally relates to a process of production of biogas from biomass and organic substrate in an anaerobic digester. In particular, the present invention relates to the monitoring and the control of such a process.

### Background Art

Anaerobic digestion also called biomethanation process is a series of processes in which microorganisms break down biodegradable material in the absence of oxygen, used for industrial or domestic purposes to manage waste and/or to release energy.

In particular, anaerobic digestion is widely used as a source of renewable energy. The process produces a biogas, which essentially comprises methane and carbon dioxide. This biogas can be used directly as cooking fuel, in combined heat and power gas engines or upgraded to natural gas quality biomethane. The use of biogas as a fuel helps to replace fossil fuels. The nutrient-rich digestate that is also produced can be used as fertilizer.

Furthermore, anaerobic digestion is particularly suited to organic material and is commonly used for effluent and sewage treatment. Anaerobic digestion is a simple process that can greatly reduce the amount of organic matter, which might otherwise be destined to be dumped at sea, landfilled or burnt in an incinerator. Almost any organic material can be processed with anaerobic digestion. Pressure from environmentally related legislation on solid waste disposal methods in developed countries has increased the application of anaerobic digestion as a process for reducing waste volumes and generating useful by-products.

However, the process of anaerobic digestion is very difficult to control. This kind of process involves the use of an anaerobic digester and the adequate digester feeding or loading rate has a decisive impact on the throughput of biomethanation process, both in case of continuously or batch-loaded digesters. Insufficient feeding of the digester will result in low methane throughput, far below the optimum achievable for a given type and size of digester and type of organic substrate or mix of substrates employed in the process. On the other hand, digester overload with biomass (overfeeding) directly affects methane/carbon dioxide ratio in produced biogas and in critical situation may easily lead to complete process extinction due to the death of microorganisms specifically involved in acetogenesis and/or methanogenesis process stages. Additionally, an optimal feeding rate of given digester is not exclusively driven by quantity of supplied biomass substrate(s) but also by its type(s). Protein, lipid, or hydrocarbon rich substrates have distinctively different digestibility rate and biomethanation potential, which needs to be taken into account when feeding. Indeed, an optimal C/N ratio of digester feed must be respected, which is pretty challenging, especially with types of substrates and composition of feed changing on weekly or even daily basis.

Most advanced digesters employ currently H₂, CH₄, NH₃, O₂, H₂S, CO₂, pH sensors. This kind of sensors is necessary to ensure a minimum of process robustness, but the information provided is neither prompt, nor exhaustive enough to ensure an optimal feeding rate and entirely secure the entire process. For example, the information provided by H₂, H₂S and pH sensors reflects a current acidity of the digestate. Unfortunately, significant rise of pH signifies that acidity of the biomass inside digester has already overcome the buffering capacity of the latter, which means that process dysfunction called "acidosis" has already occurred in the digester. Since the acidosis is lethal for bacteria driving acetogenic and methagenic reaction stages, a rapid alkalisation cure by adding of amount of HCO₃⁻ is necessary for process recovery. In case of severe digester overfeeding there is frequently no room left for economical digester recovery, even by curing with hydrogen carbonate. This results in death of populations of sensitive acetogenic and methanogenic bacteria and stops anaerobic decomposition process.

More precisely, an excess of H₂, a by-product resulting from hydrolysis and acetogenesis in overloaded digester will specifically affect acetogenic bacteria, responsible for conversion of fatty acids to acetate. Independently to H₂, an acidosis generated by the excess of fatty acids, directly hits methanogenic bacteria responsible for the final acetate conversion to biogas (methane and carbon dioxide).

Thus, when liquid phase pH drops down too much and/or too fast, the acidosis will already be too well established for an efficient recovery. As a result, a simple pH measurement is not sufficient for reliably controlling the biogas production in an anaerobic digester because when acidity establishes the buffer capacity of the system is already exhausted and it is already too late to prevent the death of the bio-system.

In view of the foregoing, there is a real need to develop a process allowing to control online and directly the production of biogas in an anaerobic digester. As a consequence, the feeding of the anaerobic digester with organic substrate could be more directly adjusted in order to avoid the overloading or under loading rates in the anaerobic digester.

### General Description of the Invention

It is an object of the present invention to provide a process for monitoring and controlling online and directly the production of biogas in an anaerobic digester allowing directly adjusting the feeding of the anaerobic digester.

The present invention therefore relates to a process for online and direct control of production of biogas in an anaerobic digester having a sludge bed and a headspace with a headspace for collecting biogas, said process comprising the steps of:
a) feeding the anaerobic digester with organic substrate,
b) extracting a sample of biogas from the headspace of the anaerobic digester,
c) analyzing the composition of the sample of biogas and detecting the quantity of one or more volatile fatty acids in said sample of biogas,
d) controlling feeding of the anaerobic digester in view of the quantity of the one or more volatile fatty acids detected in said sample of biogas.

The anaerobic digester is an apparatus suitable for implementing anaerobic digestion processes. The anaerobic digester generally comprises a heated and sealed container which is fed with organic substrate and equipped with a system (overflow or all-in all-out system) to collect the non-digested material. The anaerobic digester can be compartmented (baffled reactor) or not. It includes the continuously stirred tank reactor, the plug-flow, the lagoon types and any other reactor type allowing the anaerobic digestion of organic material for the purpose of biogas production.

The headspace of the anaerobic digester designates the superior inner part of the digester wherein the biogas is released and possibly stored. The headspace of the anaerobic digester is the inner part located above the liquid phase of the organic substrate present in the anaerobic digester. The biogas accumulates in the headspace of the anaerobic digester.

One of the main advantages of this process is that it allows avoiding the overloading and the underloading rates in the anaerobic digester. Thus, the process allows ensuring an optimal feeding rate and avoiding process dysfunction such as "acidosis".

Indeed, approaches for controlling the production of biogas in an anaerobic digester using the detection of the presence of volatile fatty acids (VFAs, mainly acetic acid, propionic acid and butyric acid) produced during the anaerobic digestion process have been presented before. In fact, the volatile fatty acids are generally considered as good indicators of the state of the process of the anaerobic digestion. Indeed, document EP1574482A1 describes an "online" capacity measurement of an anaerobic sludge bed reactor for an anaerobic wastewater purification process. In particular, document EP1574482A1 discloses that the increase of the volatile fatty acids in the effluent is a first indicator of system performance deterioration. Thus, document EP 1574482A1 suggests controlling the quantity of VFAs in the liquid effluent obtained by the process of anaerobic digestion. Furthermore, Boe et al ("An Innovative Online VFA Monitoring System for the Anaerobic Process, Based on Headspace Gas Chromatography", Biotechnology and Bioengineering, Vol 96, No. 4, March 1, 2007) also describes a method for controlling the anaerobic process by detecting VFAs in a sample of liquid from the digester.

However, both previous processes involve the sampling of liquid from the digester. Furthermore, the samples taken have to be prepared and retreated before being analyzed. Indeed, the liquid samples taken from the digester have to be filtered, concentrated, etc... before analyses can start. Document EP 1574482A1 describes that the effluent sample is analyzed by a FTIR method. FTIR method implies a preparation of the sample before the analysis. Furthermore, according to the method described in Boe et al, the samples have to be evaporated before being analyzed by gaseous chromatography (GC). Thus, both methods do not allow obtaining direct results since steps of preparation of sample are required before the analysis. Said both methods cannot be considered as "online" methods for controlling the production of biogas in an anaerobic digester. As a result, said methods do not allow to immediately react, such as by adjusting the feeding rate of the anaerobic digester in order to ensure an optimal feeding rate and to avoid process dysfunction such as "acidosis".

Contrary to the known processes involving the restrictive sampling of the liquid in the anaerobic digester and the preparation of the sample before analysis, the process according to the present invention is implemented in a very simple way. Furthermore, it allows to rapidly detect a process dysfunction from the start and accordingly to adjust without delay the feeding rate of the anaerobic digester. As a result, the process allows providing more constant conditions for the microorganisms in the anaerobic digester and avoiding the spoiling of the microorganism populations and of the feeding substrates. Thus, optimum performances are maintained for the production of biogas and decreasing of yield is avoided contrary to the known methods.

Indeed, in a surprising way, the inventors have found that monitoring the biogas composition in VFAs located in the headspace of the anaerobic digester allows for an optimized anaerobic digester feeding and thereby anticipating severe digester under- and overloading. In particular, the inventors have found that formic acid, which is the simplest VFA possessing only one Carbon (C₁), may be used as a reliable indicator of the state of the process. Formic acid is known as a precursor of H₂ production (Islam et al, 2006; Boguslaw Lupa et al., 2008) and it was described in relation with process failure when present in the feeding substrates (Hassan, M.A., Y. Shirai, N. Kusubayashi, M.I. Abdulkarim, K. Nakanishi and K. Hashimoto, 1996. Effect of Organic acid profiles during Anaerobic Treatment of Palm Oil Mill Effluent on the Production of Polyhydroxyalkanoates by Rhodobactersphaeroides. J. Ferment. Bioengin., 82(2): 151-156.), but has never been linked to process status and process monitoring. The determination of the presence of formic acid in the biogas and the variation of its concentration in relation with the process status is therefore a major progress made by the inventors.

Thus, the process proposed is based on an online monitoring of specific VFAs, in particular of formic acid, in the gas phase of the anaerobic digester headspace. VFAs are defined in the present invention as fatty acids comprising a carboxylic acid group with a carbon chain of eight carbon or fewer. Furthermore, in the context of the present invention, the term VFAs also includes lactic acid. In normal conditions these VFAs are directly or indirectly (via acetogenic reduction of H₂ and CO₂) metabolized to acetate by acetogenic bacteria but in case of process failure they accumulate in the liquid phase causing toxicity for methanogens and pH fall.

As a result, the process according to the present invention only requires the analysis of the composition of the sample of biogas in VFAs located in the headspace of the anaerobic digester without tedious preparation of the sample, such as by filtration, concentration or drying steps as for liquid samples. Indeed, said samples are already in gaseous phase.

Accordingly, another advantage of this kind of process is therefore to obtain an instantaneous and online VFAs monitoring within the gas phase. The quantity of VFAs means according to the present context either the absolute quantity of one or more VFAs or the ratiomeric quantity of one or more VFAs compared to others VFAs detected in the sample of biogas. Consequently, the feeding of the anaerobic digester can be immediately adjusted if necessary. Such access to immediate information was not possible with the previous processes and will enable an online automated process control.

A major difficulty expected to be encountered during the development of this method was the detection of chemical compounds such as VFAs present in the headspace at very low concentrations (ppb) and in the presence of gaseous background determined by few major chemical compounds (and moisture) at concentrations of several order of magnitude higher than volatile fatty acids of interest, thus masking them with standard and not filtered gas phase detection methods. Indeed, the generated biogas is composed of a large portion of methane (about 60 %), carbon dioxide (about 40%), H₂S and NH₃ (few %) and humidity (few %) but also a large number of other organic compounds which chemical nature and respective concentrations are low and vary depending on the feeding regime of the digester. Furthermore, it is known by the skilled person that at neutral or basic pH, the VFAs, which are weak acids, are retained in the liquid of the digester by the ammonia. Indeed, biogas composition analysis achieved on biogas plants by gas chromatography do not yield any VFA (Kymäläinen et al., 2012, Biogasification of biowaste and sewage sludge - Measurement of biogas quality, Journal of Environmental Management 95: S122-S127; Rasi S. 2009., Biogas composition and upgrading to biomethane, Doctoral thesis; Jyväskylä studies in Biological and Environmental Science, vol. 202, University of Jyväskylä, Finland. 76pp.; Rasi et al. 2011. Trace compounds affecting biogas energy utilization - A review. Energy conversion and management; 52: 3369-3375, Arnold and Kajolinna. 2010, Development of on-line measurement techniques for siloxanes and other trace compounds in biogas, Waste Management 30: 1011-1017). The VFAs are potentially released in the biogas of the digester only when the pH of liquid becomes acid or if the digestate sample is heated as an external pretreatment. However, when acid pH is reached by the system, it is already too late for controlling the process of production of biogas since the acidosis will already be too well established for an efficient recovery. Especially undissociated acids (R-COOH) have an inhibiting effect as they penetrate as lipophilics into microbial cells, where they denaturate the cell proteins. Moreover, the undissociated forms prevail at pH<7 and the inhibition is intensified by the drop in the pH-value (Deublein and Steinhauser. 2008. Biogas from waste and renewable resources. Wiley-VCH Verlag GmbH & Co. KGaA, Weinheim, Germany). When ammonia concentration in the digestate induces pH-value >7, the VFAs occur in the dissociated form (R-COO- + H⁺) and are retained in the liquid digestate as ammonium salt (R-COONH₄). It was believed that the VFAs become detectable in a sufficient quantity only when the system reaches the acidosis state. Thus, the man skilled in the art would not have considered detecting VFAs in the biogas since the release of a detectable amount of VFAs in biogas was regarded as being synonymous with acidosis state of the digester. So the live and online detection of VFAs in trace level (ppb) in biogas constitutes a major innovation. Once more, an unexpected innovation is the possibility to measure such trace compounds without the need for a pretreatment or additional sample preparation either tending to increase their concentration in the gas phase sample (chemical extraction and evaporation from liquid sample or compound-specific pre-concentration) or to filter out principal biogas constituents shielding the VFAs of interest.

Thus, the present invention allows anticipating severe digester overloading that may result in critical acidosis, optimizing digester feeding and *in fine* improving methane production through a smoother process control.

### Description of Preferred Embodiments

As already mentioned, the present invention relates to a process for online and direct control of production of biogas in an anaerobic digester comprising the steps of:
a) feeding the anaerobic digester with organic substrate,
b) extracting a sample of biogas from the headspace of the anaerobic digester,
c) analyzing the composition of the sample of biogas and detecting the quantity of one or more volatile fatty acids in said sample of biogas,
d) controlling feeding of the anaerobic digester in view of the quantity of the one or more volatile fatty acids detected in said sample of biogas.

According to a further embodiment of the present invention, the volatile fatty acids are selected from formic acid, acetic acid, lactic acid, propionic acid, butyric acid, isobutyric acid, valeric acid, isovaleric acid, caproic acid, heptanoic acid, caprylic acid or 2-ethylhexanoic acid; or two or more thereof. It means that the process involves the detection of one or several volatile fatty acid(s) selected from the group consisting in: formic acid, acetic acid, lactic acid, propionic acid, butyric acid, isobutyric acid, valeric acid, isovaleric acid, caproic acid, heptanoic acid, caprylic acid and 2-ethylhexanoic acid.

In particular, the preferred volatile fatty acids are formic acid, valeric acid, propionic acid, caproic acid, butyric acid, or isovaleric acid. In other words, the process involves the detection of one or several volatile fatty acid(s) selected from the group consisting in formic acid, valeric acid, propionic acid, caproic acid, butyric acid, and isovaleric acid.

According to another embodiment, the volatile organic compound is most preferably formic acid. Indeed, among the VFAs detected by the present process, formic acid appears to be one of the earliest gas phase indicators of ongoing process dysfunction such as acidosis and it was not described previously as an indicator of process state.

Similarly to acidosis, an excess of nitrogen in the feeding ratio of the digester causes a dysfunction known as ammonia toxicity or alkalosis. In this case, VFAs also accumulate but the drop of pH is compensated by the strong alkalinity of NH₃ that eventually leads to pH rise. Typical symptoms of alkalosis are high concentration of VFAs in digestate, high NH₃ concentration (above 3kg NH₃/m³ digestate) and pH close or above to 8. These parameters also alter strongly the biogas throughput and its composition. Thus, according to one embodiment, the process also comprises the step of detection of the quantity of NH₃ in the sample of biogas extracted from the headspace of the anaerobic digester.

According to one embodiment of the present invention, the process may include the detection and the analysis of other natural Volatile Organic Compounds (VOCs) in addition to the VFAs. Natural VOCs, in addition to VFAs, include volatile organic molecules such as ethanol, alkane, alkene, and ketones which are release during process changes and play an important role in the pattern-matching discrimination of the process status. In particular, natural VOCs do not include silanes or siloxanes.

The analysis of sample of biogas is more preferably carried out by a detector which comprises an Ion Mobility Spectrometer (IMS).

Said IMS may be chosen among a time-of-flight ion mobility spectrometer, a high Field Asymmetric waveform Ion Mobility Spectrometry (FAIMS) or a Differential ion Mobility Spectrometer (DMS).

In particular the high Field Asymmetric waveform Ion Mobility Spectrometry or the Differential ion Mobility Spectrometer is a Micro-Electro-Mechanical System (MEMS) detector "chip" based on silicon or ceramics substrates.

In another embodiment, the analysis of sample of biogas may be employed by half-analytical detection of specific VFAs. Said method is employed in situations when a precise single-peak attribution and quantification is not possible or not suitable. By selecting a spectral area of interest, for example a rectangle given by gas chromatography retention times (t ret-δ, t ret+δ) and compensation voltages (Vc-s, Vc+s) for the fixed value of radiofrequency dispersion field VRF, one may encompass the influence of current gas background on peak position. One may also include both, monomer and dimer peaks in one selected area.

In a still further embodiment, the analysis of sample of biogas may be made by pattern-matching discrimination. This approach is the most suitable in situations where precise peak identification is not possible, for example due to the complexity and variance of gas background. It may also be used in parallel to the two previous methods as increasing the confidence level of the decision. This approach requires a number of representative samples of the main stages of the digester, described by few macroscopic process figures of merit like methane/carbon dioxide ratio and volume of methane produced from 1 kg of given substrate.

According to a still further embodiment of the present invention, it further comprises a concomitant process of diluting in air with the extraction of the sample, preferably in synthetic air. The dilution with air is performed simultaneously with the extraction of the sample from the headspace. It means that the dilution of the sample occurs in the same time than sampling the biogas thus, it does not cause any delay or additional preparation of the sample prior to the analysis. The term "synthetic air" in the present context indicates a mixture of about 21 % of O₂ and 79% of N₂.

Indeed, it has been shown by the inventors that dilution of biogas sample in air leads to the increase of ion clusters formation advantageously compensating and overcoming the reduction of volume concentration of the analyte in sampled probe. Thus, even though the physical analyte concentration is lower than in the original biogas, the detection sensitivity is highly improved thanks to the increased number of ion clusters engaging the analytes of interest.

In addition, the presence or increase of oxygen molecules (usually almost not present in the biogas sample) in the target gas sample leads to the formation of negative reactant ions (monomer: MO₂-(H₂O)ₙ dimer: M₂O₂-(H₂O)ₙ₋₁), enabling the appearance of the negative part of the DMS spectrum. This phenomenon was again not expected. Diluting the biogas with air or synthetic air improves the detection of compounds present as traces, whereas the contrary would have been expected: dilution would lead to weakest detection of the trace compounds. Dilution also greatly facilitates the attribution, and furthermore, a precise time tracking of (tiny) VFAs peaks in very complex DMS spectrum mostly populated by primary gas compounds at much higher concentrations. Furthermore, the dilution of sample of biogas in (synthetic) air may be carried out at pressures between 1.025 and 1.1 Atm.

According to one embodiment, the process of the present invention does not include any additional steps of preparation of the sample of biogas before the analysis of step c). It means that the process according to the present invention does not include additional steps such as filtration, drying, concentration of the sample, etc... prior to the analysis of the sample.

Although not necessary, a process according to present invention may also comprise several additional steps in order to optimize the results obtained. Said additional steps are for example the reduction of the moisture in the sample of biogas extracted from the headspace of the anaerobic digester, the preconcentration of sample of biogas, and the separation of analyte.

The amount of moisture in the sample of biogas may be reduced by using a moisture condenser after step b) of extracting a sample of biogas produced in headspace and prior to step c) of analyzing the composition of said sample of biogas. Furthermore the moisture condenser may be selected among the group constituted by a stainless steel tube, silica desiccant and hydrophilic material. The moisture content in the sample of biogas may be detected by any suitable moisture sensor.

Furthermore, a preconcentration of sample of biogas may be optionally carried after step c) of analyzing the composition of the sample of biogas extracted from the headspace of anaerobic digester and prior to step d) of controlling feeding of the anaerobic digester in view of the quantity of VFAs detected in said sample of biogas. The step of preconcentration may be ensured by preconcentrators designed in MEMS (Micro-Electro-Mechanical Systems) technology and filled with specific adsorbent selected among the group constituted by activated unstructured carbons, carbon nanotubes, microporous aluminosilicates or zeolites, ceramic beads, sorbative polymers. Furthermore, the preconcentrator is optionally associated to a high yield heating resistance. Said high yield heating resistance heats up the volatile organic compounds to the temperature of 100°C-500°C, preferably about 350°C. Moreover, the temperature is set by a temperature Control Unit usually constituted by a micro-processor electronics providing a precise control of entire operation sequence of the complete system.

The sample of biogas is submitted to an analyte separator after the steps of preconcentration and heating and prior to step d) of analyzing the composition. Said analyte separator is selected among the group constituted by a single or multiple filtering membrane, a single thread gas chromatography column or a bundled fibers or micro-machined multicapillary column.

Optionally, the process may also comprise a step of cleaning and rinsing the preconcentrator, the analyte separator and the detector with neutral carrier gas after step d) of analyzing the composition of sample of biogas.

According to a preferred embodiment of the present invention the headspace gas is sampled through the outlet of the digester, preferably equipped with water condensation circuit in order to reduce the sample saturation with moisture. A Differential ion Mobility Spectrometer (DMS) or high Field Asymmetric waveform Ion Mobility Spectrometer (FAIMS) is coupled to 10m chromatographic columns with cleaned and dried air used as eluting gas. Headspace sample is flowing into atmospheric purge vessel, from which the control system is pumping out the sample during the sampling time ranging from 15 seconds to 180 seconds and streaming it to the carbon-filled preconcentrator. The latter is heated up to 300°C during the flash desorption into the GC column. By closing an entry valve, one may omit both, preconcentrator and column, and generate a complementary 3D dispersion plot (VRF, VC, I). After every measurement, the air inside the installation is recycled through the cleaning system comprising carbon and molecular sieve scrubbers. The gas sample may be either analyzed by on-site & online FAIMS or GC-FAIMS analyzer, or collected and transported into impermeable bags, metal gas reservoirs or large gas-phase syringes and then analysed out of biogas manufacturing site.

All the embodiments disclosed hereinabove might be combined where deemed necessary and within reason.

### Brief Description of the Drawings

Preferred embodiments of the invention will now be described, by way of example, with reference to the accompanying drawings in which:

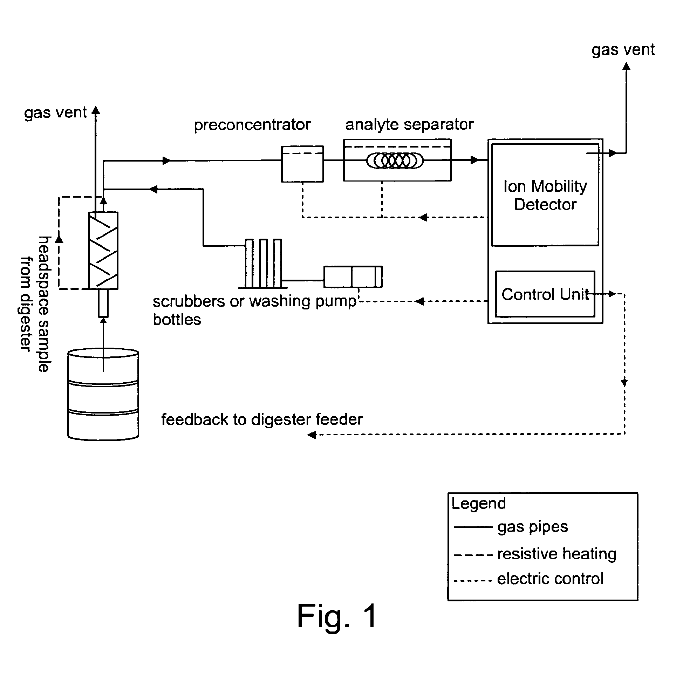

Fig. 1 is a block schematic diagram of one embodiment of the present invention showing an automated process. First, a sample of biogas is extracted from the headspace of an anaerobic digester and is caused to flow into atmospheric purge vessel. A part of the biogas extracted from the headspace of the anaerobic digester may also be evacuated through a gas vent of the atmospheric purge vessel. Another part of the sample of biogas is caused to flow to a preconcentrator through gas pipes. Then, the sample of biogas is caused to flow through gas pipes to an analyte separator. The preconcentrator and the analyte separator also comprise a heating resistance in order to warm the sample of biogas extracted. Said heating resistances are controlled by the Control Unit. Following, the steps of preconcentration and separation of analytes, the sample of biogas is then submitted to an Ion Mobility Detector (such as Ion Mobility Spectrometer) for detecting the quantity of VFAs in sample of biogas. The Ion Mobility Detector also comprises a gas vent for allowing the sample of biogas to be evacuated after the analysis. In regard of the quantity of VFAs detected in sample of biogas, the Control Unit adjusts automatically if necessary, by electric control, the feeding rate of anaerobic digester. The Control Unit also electrically controls the step of cleaning the preconcentrator, the analyte separator and the Ion Mobility Detector. Thus, after each analysis of a sample of biogas, the preconcentrator, the analyte separator and the Ion Mobility Detector are cleaned with neutral carrier gas provided by scrubbers or washing pump bottles.

Further details and advantages of the present invention will be apparent from the following detailed description of several not limiting embodiments.

### Example: Detection of the VFAs including formic acid in mini-digesters

1. Setup of test of mini-digesters

2000 ml total capacity polypropylene polyethylene test digesters equipped with continuous feeding tubes were employed and placed in thermal bath at constant 37°C. They were fitted with auxiliary sensors allowing periodic estimation of the biogas throughput, of the methane to carbon dioxide ratio and of the pH. Two digesters were used in parallel, both initially loaded with 1600 ml anaerobic waste water treatment sludge, 1g of glucose and 1g of the alimentary rape oil. After initiation of the digestion, the first mini-digester was daily fed with constant quantity of 1g of 1:1 glucose / oil mixture and was used as reference. The second one was progressively loaded with 1 g, 1.5g, 2g, 2.5g, 3g, 10g and 16g of the same substrate mix. Besides October 30^{th} - November 1^{st} 2010 and following weekend (November 11^{th}-12^{th}), both digesters were fed once a day.

2. Detection trials in the digester headspace

2.1 General

All spectra have been collected within a 30 seconds sampling time in so-called (DMS) scanning mode experiments. The sample accumulated in the carbon preconcentrator was flash-desorbed in the 37°C - 250°C temperature range and streamed to the XLB-type, 10m long, analyzer built-in GC column. GC thermal profile covering 40°C - 150°C temperature range with heating ramp of 20°C/min was found optimal for the separation of various VFAs, including isomers.

DMS scanning mode experiments were conducted at fixed value of the radio frequency pulsed dispersion field (22kV/cm or VRF=1100V) and with compensation voltage swapping from -30V to +10V. Spectra have been typically recorded 12h after digester loading. It typically took less than 7 min per run, including analyzer self-cleaning cycle. The full experiment lasted from October 26^{th} 2010 (first data collection after initial substrate loading on October 25th) to November 11^{th} 2010 (#17 day) and yielded #12 measurement points. No data was collected on October 30^{th}, 31^{st} and November 1^{st}, 6^{th} and 7^{th}.

Biogas samples were collected in the external three layers PET/aluminium/PET collection bags and stored a few hours before GC/DMS data collection via included valve. For that reason, all GC/DMS data should be rather seen as the effect of modification of biological activity over the period of filling of the bag rather than in situ nascendi measurements. In parallel to the CG/DMS spectra, the digestate's pH, the amount of produced biogas (gas counter of the humid drum type) and methane / carbon dioxide proportions were measured by auxiliary gauges (dedicated NIR sensors).

The qualitative and quantitative differences in between different sets of data collected at different moments are clearly perceptible on the resulting 3D scanning plots (VC-compensation voltage (V), GC-retention time (s), peak intensity (V)). The first concerns peak presence and distribution over monomer and/or dimer product ion(s), while the second deals with peak intensities of specific monomer or dimmer peaks as resulting from the specific VFA's ion abundances in the digester's headspace. Indeed, the co-existence and intensity ratios of monomer and dimer peaks intrinsically hold the information on compound concentration. In case of low analyte concentration, only monomer product ions are observed whilst at higher analyte concentrations, depleted reactant ions (RIP) give rise also to positive or negative ion product dimers, depending to the analyte's chemical nature. In case of volatile fatty acids being the main analytical targets of present digester control methods, both positive and negative dimers (Positive dimer: M₂H⁺(H₂O)ₙ₋ₓ and negative dimer: M₂O₂⁻(H₂O)ₙ₋ₓ with M the volatile fatty acid and n-x the number of water molecules) may be observed.

2.2 Time evolution of VFAs

The identified VFAs's peaks have been followed over entire experiment duration over 17 days. Their evolution was aligned with digester loading profile, pH change and estimated methane throughput. It comes out that in the period from November 3^{rd} to November 5^{th}, the methane output reaches its maximum (3343ml on November 5^{th}) before starting to fall down, along with a sharp pH drop. A deliberately excessive daily feeding of 10g, 16g and 16g substrate loads over those 3 days lead to the further digestate acidosis.

Valeric, propionic, caproic and formic acids turn out to be the most responsive to almost every digester-feeding event. Their respective peaks (proportional to the ion abundance) are also the most changing during the critical overfeed period from October 3^{rd} - October 5^{th}, i.e. in between of CH₄ optimum (maximum) and the beginning of the acidosis. As shown in the Table 1 below, a 60% digester overload causes a nearly 5-fold change in observed peak intensity of the valeric acid and 2.5 fold change of the propionic acid.

**Table 1. Quantitative, peak amplitude change within a 3 days period for adequate feeding to 60% overfeeding (October 2010)**

| loading (g) | acetic | formic | propionic | butyric | isobutyric | valeric | isovaleric | caproic | heptanoic | pH | Biogas vol (mL) | CH₄ vol (mL) |
|---|---|---|---|---|---|---|---|---|---|---|---|---|
| 60% | -39% | 129% | 262% | -1% | 56% | 503% | -10% | 219% | 26% | -10% | 47% | 20% |

Said results show that low molar mass volatile fatty acids (formic, propionic, (iso)butyric, (iso)valeric, caproic, heptanoic) are detectable by gas chromatography/differential ion mobility spectrometry in the biogas of the headspace because of their high volatility easing the transfer of these compounds from the liquid phase to the headspace and its low ionization energy which increases their probability of being ionized and therefore of being detected.

### 2.3 VFAs's correlation to principal macroscopic process indicators

One may ask the question of the cross-dependency of VFAs's intensities to the principal process indicators like pH, methane / carbon dioxide ratio in produced biogas and total methane volume. Thus, Pearson's correlation moments are given over three different observation periods in Table 2 below.

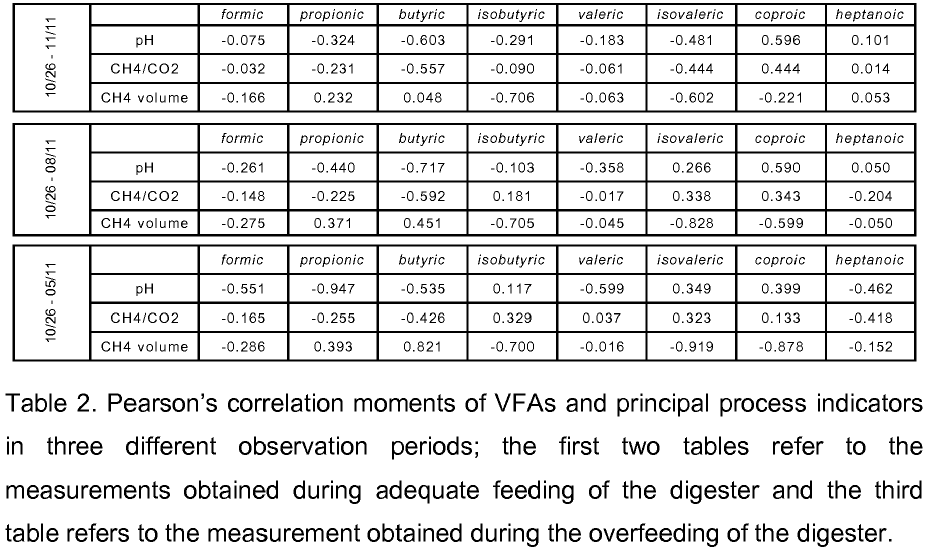

The upper part of the table yields the correlations computed over the full duration of the experiment (26/10/2010 - 11/11/2010), the middle one (26/10/2010 - 08/11/2010) restraints to the period when the digester was still daily loaded, while the bottom, covers exclusively the period (26/10/2010 - 05/11/2010) of the digester performance during overfeeding, when it turned to the severe acidosis. Sensitivity to acidosis varies among digesters, the literature often refers to a VFAs concentration of 3000mg/l acetic acid equivalent as a threshold limit towards high risk of VFAs intoxication. However, with high ammonia concentration in the digestate, digesters can stand much higher VFA concentrations (up to 15000mg/l have been observed in efficient digesters with ammonia concentration exceeding 3 kg N-NH₃ kg⁻¹ of digestate). In the present approach a relative increase in formic (1.3 fold), propionic (2.6 folds), and valeric (5 folds) acids in the biogas was considered as a good indication of process shift towards acidosis.

The results show that correlations are higher when the last three days (no more feeding and low pH and almost inhibited methane production indicating a severe microbial disorder) are not included in the analysis. It suggests, that during the "non critical" digester feeding, several VFAs are related to the main process indicators and therefore may be used as process triggers. The data once more points out the different behaviour of the butyric acid (positively correlated to methane volume), while all others (if significant), isobutyric, isovaleric and caproic acids are negatively correlated to the methane. Formic acid is a good candidate as the macroscopic process indicators because of its high volatility allowing immediate exchange between the liquid phase and the headspace of the digester and thus a smooth control of biomethanation process and a fair correlation with pH.

VFAs evaporated from the liquid phase of the digestate appear in the headspace concentrations fair enough to be detected. They definitely follow microbiologically driven variations of the bioprocess.

## Claims

1. Process for online and direct control of production of biogas in an anaerobic digester with a headspace for collecting biogas, said process comprising the steps of:
a) feeding the anaerobic digester with organic substrate,
b) extracting a sample of biogas from the headspace of the anaerobic digester,
c) analyzing the composition of the sample of biogas and detecting the quantity of one or more volatile fatty acids in said sample of biogas,
d) controlling feeding of the anaerobic digester in view of the quantity of the one or more volatile fatty acids detected in said sample of biogas.

2. Process according to claim 1 wherein the volatile fatty acids are selected from formic acid, acetic acid, lactic acid, propionic acid, butyric acid, isobutyric acid, valeric acid, isovaleric acid, caproic acid, heptanoic acid, caprylic acid or 2-ethylhexanoic acid; or two or more thereof.

3. Process according to claims 1 or 2 wherein the volatile fatty acids are valeric acid, propionic acid, caproic acid, butyric acid, isovaleric acid or formic acid.

4. Process according to any one of claims 1 to 3 wherein the volatile fatty acid is formic acid.

5. Process according to any one of claims 1 to 4 wherein the analysis of step c) is carried out by a detector which comprises an Ion Mobility Spectrometer (IMS).

6. Process according to claim 5 wherein the Ion Mobility Spectrometer is a time-of-flight ion mobility spectrometer, a high Field Asymmetric waveform Ion Mobility Spectrometry (FAIMS) or a Differential ion Mobility Spectrometer (DMS).

7. Process according to claim 6 wherein the high Field Asymmetric waveform Ion Mobility Spectrometry or the Differential ion Mobility Spectrometer is a Micro-Electro-Mechanicals System (MEMS) detector "chip" based on silicon or ceramics substrates.

8. Process according to any one of claims 1 to 7 **characterized in that** it further comprises a concomitant process of diluting in air the sample of biogas when it is extracted from the headspace of the anaerobic digester.

9. Process according to any one of claims 1 to 8 **characterized in that** it does not include any additional steps of preparation of the sample before the analysis of step c).
